(19) Europäisches Patentamt
European Patent Office
Office européen des brevets

(11) EP 0 919 217 A1

(12) **DEMANDE DE BREVET EUROPEEN**

(43) Date de publication:
**02.06.1999 Bulletin 1999/22**

(51) Int. Cl.6: **A61K 7/00**, A61K 7/48

(21) Numéro de dépôt: **98401443.1**

(22) Date de dépôt: **12.06.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **08.07.1997 FR 9708673**

(71) Demandeur: **L'OREAL**
**75008 Paris (FR)**

(72) Inventeurs:
- **Favre, Sophie**
  **94550 Chevilly-Larue (FR)**
- **Terren, Nadia**
  **92340 Bourg la Reine (FR)**

(74) Mandataire: **Lhoste, Catherine**
**L'OREAL,**
**D.P.I.,**
**6, rue Bertrand Sincholle**
**92585 Clichy Cédex (FR)**

(54) **Composition à base de vesicules lipidiques et d'un poly (acide 2-acrylamido 2-methylpropane sulfonique) reticulé et neutralisé à au moins 90%**

(57) La présente invention se rapporte à une composition cosmétique et/ou dermatologique comprenant une dispersion aqueuse de vésicules lipidiques contenant au moins un polymère poly(acide 2-acrylamido 2-méthylpropane sulfonique) réticulé et neutralisé à au moins 90%. Il comprend en général, distribués de façon aléatoire :

a) de 90 à 99,9% en poids de motifs de formule générale (1) suivante :

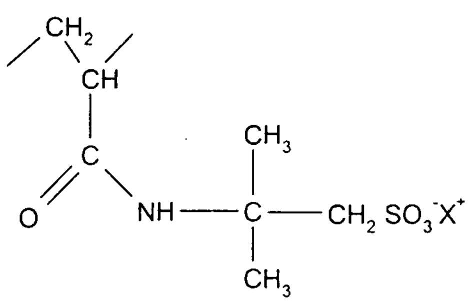

(1)

dans laquelle $X^+$ désigne un cation ou un mélange de cations, au plus 10% mol des cations $X^+$ pouvant être des protons $H^+$ ;
b) de 0,01 à 10% en poids de motifs réticulants provenant d'au moins un monomère ayant au moins deux doubles-liaisons oléfiniques ; les proportions en poids étant définis par rapport au poids total du polymère.

EP 0 919 217 A1

## Description

La présente invention se rapporte à une composition comprenant une dispersion aqueuse de vésicules lipidiques et un polymère poly(acide 2-acrylamido 2-méthyl-propane sulfonique) réticulé et neutralisé à au moins 90%. Elle concerne également ses utilisations en application topique.

Les composés acides actifs sur le plan cosmétique ou dermatologique tels que les hydroxyacides, les $\alpha$- et $\beta$-cétoacides sont de plus en plus utilisés dans le domaine cosmétique pour le soin du visage et/ou du corps. En particulier, les hydroxyacides sont utilisés plus spécialement pour donner au visage un teint lumineux et éclatant et donc une bonne mine, un aspect lisse et plus jeune, pour le traitement cosmétique des rides et/ou des ridules de la peau ainsi que pour faire disparaître les comédons dus à l'acné.

Les compositions classiquement utilisées dans les domaines cosmétique et/ou dermatologique sont des émulsions eau-dans-huile (E/H), des émulsions huile-dans-eau (H/E) ou des gels aqueux, dans lesquels il est souvent difficile, voire même impossible d'incorporer des actifs acides organiques comme l'acide kojique, l'acide caféique, l'acide salicylique et ses dérivés.

En général, ces actifs acides ont tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de ces compositions à traitement spécifique.

Pour solubiliser certains de ces actifs, il est connu d'utiliser des émulsions E/H ou H/E dans lesquelles la phase aqueuse présente un pH acide. Pour que ces émulsions soient stables (non séparation des phases aqueuse et huileuse), il est nécessaire d'utiliser des émulsionnants (ou tensioactifs). Malheureusement, ces tensioactifs sont souvent irritants pour la peau. En outre ces émulsions manquent souvent de fraîcheur à l'application, ce qui peut gêner leurs utilisations pendant les périodes chaudes de l'année et/ou dans les pays chauds. Un gel aqueux est beaucoup plus apprécié dans ces conditions d'utilisation. Mais sa trop grande quantité d'eau ne permet pas d'y introduire les actifs présentant un certain caractère lipophile. La stabilité de ces gels est par ailleurs médiocre.

Il subsiste donc le besoin d'une composition stable et utilisable dans les domaines cosmétique et/ou dermatologique permettant une solubilisation suffisante des actifs acides généralement utilisés dans ces domaines en vue d'une efficacité maximum. D'autre part, on recherche également en cosmétique une viscosité suffisamment élevée pour faciliter la prise du produit hors de son conditionnement sans perte significative, imiter la diffusion du produit à la zone locale de traitement et pouvoir l'utiliser dans des quantités suffisantes pour obtenir l'effet cosmétique ou dermatologique recherché. Cet objectif est important pour les formulations telles que celles des produits pour le soin, l'hygiène ou le maquillage qui doivent bien s'étaler de façon homogène sur la surface locale à traiter. Pour satisfaire à ces conditions, on augmente généralement la viscosité des compositions par l'ajout de polymères épaississants et/ou gélifiants.

On connaît dans la demande de brevet EP-A-0 728 459 des formulations acides stables comprenant ce type d'actif, sous la forme d'une dispersion huile-dans-eau comprenant des vésicules lipidiques agissant comme dispersant de l'huile dans la phase continue aqueuse. Ces formulations présentent des qualités d'application, de maquillage et de confort spécifiques aux formulations ayant pour base des systèmes vésiculaires. Pour obtenir une consistance satisfaisante pour une application confortable et une activité efficace, on incorpore généralement dans ces formulations des polymères gélifiants hydrosolubles classiques tels que les dérivés de cellulose, les gommes naturelles et des polymères synthétiques comme les Carbopol et plus particulièrement le mélange de polyacrylamide isoparaffine, alcool laurique oxyéthyléné à 7 moles d'oxyde d'éthylène vendu sous le nom SEPIGEL 305 par la Société SEPPIC.

Les épaississants du type CARBOPOL sont généralement incompatibles avec les actifs acides et ne conduisent à la consistance souhaitée. Les gommes naturelles comme les gommes de cellulose ou les gommes de Xanthane, conduisent le plus souvent à des formulations poisseuses voire collantes.

Le mélange de polyacrylamide, isoparaffine, alcool laurique oxyéthyléné à 7 moles d'oxyde d'éthylène vendu sous le nom SEPIGEL 305 par la Société SEPPIC présente l'inconvénient de contenir des tensio-actifs ayant un potentiel irritant vis-à-vis de la peau. De plus, les formulations vésiculaires contenant cet épaississant ont tendance en fin d'application sur la peau à donner une sensation légèrement collante.

La demanderesse a découvert de manière surprenante une nouvelle famille de polymères gélifiants sans tensio-actif permettant d'obtenir des formulations cosmétiques et dermatologiques à base de vésicules lipidiques sous forme de dispersons huile-dans-eau stables et homogènes permettant de résoudre les inconvénients évoqués ci-dessus et notamment de solubiliser et de stabiliser les actifs organiques acides à des pH inférieurs ou égal à 5. D'autre part, les dispersions obtenues selon l'invention sont de façon inattendue sensiblement plus fines que celles de l'art antérieur et présentent un étalement sur la peau plus facile et plus confortable.

Ainsi, l'invention a pour objet une composition cosmétique et/ou dermatologique contenant une dispersion aqueuse de vésicules lipidiques et au moins un poly(acide 2-acrylamido 2-méthylpropane sulfonique) réticulé et neutralisé à au moins 90%.

Un autre objet de l'invention consiste une composition cosmétique et/ou dermatologique contenant une dispersion aqueuse de vésicules lipidiques caractérisée par le fait qu'elle contient au moins un composé acide conférant à ladite dispersion un pH acide et au moins un poly(acide 2-acrylamido 2-méthylpropane sulfonique) réticulé et neutralisé à au moins 90%.

Les polymères poly(acide 2-acrylamido 2-méthylpropane sulfonique) réticulés et pratiquement ou totalement neutralisés, conformes à l'invention, sont hydrosolubles ou gonflables dans l'eau. Ils sont en général caractérisés par le fait qu'ils comprennent, distribués de façon aléatoire :

a) de 90 à 99,9% en poids de motifs de formule générale (1) suivante :

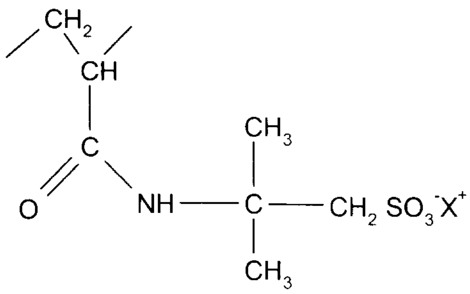

(1)

dans laquelle $X^+$ désigne un cation ou un mélange de cations, au plus 10% mol des cations $X^+$ pouvant être des protons $H^+$;

b) de 0,01 à 10% en poids de motifs réticulants provenant d'au moins un monomère ayant au moins deux doubles-liaisons oléfiniques ; les proportions en poids étant définis par rapport au poids total du polymère.

Ainsi, les polymères de l'invention sont des homopolymères, c'est-à-dire des polymères obtenus par polymérisation d'un monomère, ce monomère étant dans la présente invention les motifs de formule (1).

De façon préférentielle, les polymères de l'invention comportent un nombre de motifs de formule (1) dans une quantité suffisamment élevée pour obtenir un volume hydro-dynamique du polymère en solution d'eau ayant un rayon allant de 10 à 500 nm, de distribution homogène et unimodale.

Les polymères selon l'invention plus particulièrement préférés comprennent de 98 à 99,5 % en poids de motifs de formule (1) et de 0,2 à 2 % en poids de motifs réticulants.

$X^+$ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations $NH_4^+$ et 0 à 10% mole sont des protons ($H^+$).

Les monomères de réticulation ayant au moins deux doubles-liaisons oléfiniques sont choisis par exemple parmi le dipropylèneglycol-diallyléther, les polyglycoldiallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles-liaisons oléfiniques sont plus particulièrement choisis parmi ceux répondant à la formule générale (2) suivante :

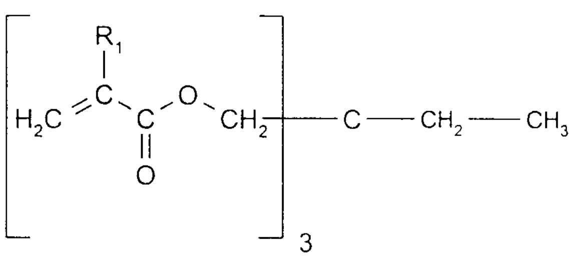

(2)

dans laquelle $R_1$ désigne un atome d'hydrogène ou un alkyle en $C_1$-$C_4$ et plus particulièrement méthyle (triméthylol propane triacrylate).

La réaction de polymérisation des polymères de l'invention produit non seulement des chaînes linéaires mais aussi des molécules de polymère ramifiées ou réticulées. Ces molécules peuvent être caractérisées notamment par

leur comportement rhéologique dans l'eau mais plus particulièrement par la diffusion de la lu mière dynamique.

Dans le cas de la caractérisation des molécules par la diffusion de la lumière dynamique, on mesure la distribution du volume hydrodynamique des structures du polymère. Les macromolécules dissoutes dans l'eau sont flexibles et entourées par une enveloppe de solvatation formée de molécules d'eau. Avec des polymères chargés comme ceux de l'invention, la taille des molécules dépend de la quantité de sel dans l'eau. Dans les solvants polaires, la charge uniforme le long de la chaîne principale du polymère conduit à une expansion importante de la chaîne polymérique. Le fait d'accroître la quantité de sel augmente la quantité d'électrolyte dans le solvant et écrante les charges uniformes du polymère. En plus des molécules transportées dans l'enveloppe de solvatation, les molécules de solvant sont fixées dans les cavités du polymère. Dans ce cas, les molécules de solvant font partie des macromolécules en solution et se déplacent à la même vitesse moyenne. Ainsi, le volume hydrodynamique décrit la dimension linéaire de la macromolécule et de ces molécules de solvatation.

Le volume hydrodynamique $v_h$ est déterminé par la formule suivante :

$$v_h = M/N_A \times (V_2 + dV_1)$$

avec :

M désignant la masse en grammes de la macromolécule non-dissoute ;
$N_A$ désignant le nombre d'Avogadro ;
$V_1$ désignant le volume spécifique du solvant ;
$V_2$ désignant le volume spécifique de la macromolécule ;
d la masse en grammes du solvant qui est associé avec 1 gramme de macromolécule non-dissoute.

Si la particule hydrodynamique est sphérique, il est alors facile de calculer à partir du volume hydrodynamique le rayon hydrodynamique par la formule :

$$V_h = 4\Pi R^3/3$$

avec R désignant le rayon dynamique.

Les cas où les particules hydrodynamiques sont des sphères parfaites sont extrêmement rares. La majorité des polymères synthétiques impliquent des structures compactées ou des ellipsoïdes à haute excentricité. Dans ce cas, la détermination du rayon s'effectue sur une sphère qui est équivalente d'un point de vue frottement à la forme de la particule considérée.

En règle générale, on travaille sur des distributions de poids moléculaire et donc sur des distributions de rayon et de volume hydrodynamique. Pour les systèmes poly-dispersés, on doit calculer la distribution des coefficients de diffusion. De cette distribution, on en déduit les résultats relatifs à la distribution radiale et à la distribution des volumes hydrodynamiques.

Les volumes hydrodynamiques des polymères de l'invention sont en particulier déterminés par diffusion de la lumière dynamique à partir des coefficients de diffusion D selon STOKES-EINSTEIN de formule : $D = kT/6\Pi\eta R$ où k est la constante de Boltzmann, T la température absolue en degrés Kelvin, $\eta$ est la viscosité du solvant (eau) et R est le rayon hydrodynamique.

Ces coefficients de diffusion D sont mesurés selon la méthode de caractérisation d'un mélange de polymères par diffusion au LASER décrite dans les références suivantes :

(1) Pecora, R ; Dynamic Light Scattering ; Plenium Press, New York, 1976 ;
(2) Chu, B ; Dynamic Light Scattering ; Academic Press, New York, 1994 ;
(3) Schmitz, KS ; Introduction to Dynamic Light Scattering ; Academic Press, New York, 1990 ;
(4) Provincher S.W. ; Comp. Phys., 27, 213, 1982 ;
(5) Provincher S.W. ; Comp. Phys., 27, 229, 1982 ;
(6) ALV Laservertriebgesellschaft mbH, Robert Bosch Str. 47, D-63225 Langen, Germany ;
(7) ELS-Reinheimer Strasse 11, D-64846 Gross-Zimmern, Germany ;
(8) CHI WU et al , Macromolecules, 1995, 28,4914-4919.

Les polymères particulièrement préférés sont ceux présentant une viscosité mesurée au viscosimètre BROOKFIELD dans une solution d'eau à 2% et à 25 °C supérieure ou égale à 1000 cps et plus préférentiellement allant de 5000 à 40000 cps et plus particulièrement de 6500 à 35000 cps.

Les poly(acide 2-acrylamido 2-méthylpropane sulfonique) réticulés de l'invention peuvent être obtenus selon le procédé de préparation comprenant les étapes suivantes :

(a) on disperse ou on dissout le monomère acide 2-acrylamido 2-méthylpropane sulfonique sous forme libre dans une solution de tertio-butanol ou d'eau et de tertio-butanol;

(b) on neutralise la solution ou la dispersion de monomère AMPS obtenue en (a) par une ou plusieurs bases minérales ou organiques, de préférence l'ammoniaque $NH_3$ , dans une quantité permettant d'obtenir un taux de neutralisation des fonctions acides sulfoniques du polymère allant de 90 à 100% ;

(c) on ajoute à la solution ou dispersion obtenue en (b) le ou les monomères réticulants ;

(d) on effectue une polymérisation radicalaire classique en la présence d'amorceurs de radicaux libres à une température allant de 10 à 150°C : le polymère précipitant dans la solution ou la dispersion à base de tertio-butanol.

Les poly(acide 2-acrylamido 2-méthylpropane sulfonique) réticulés, pratiquement ou totalement neutralisés sont présents dans les compositions cosmétiques ou dermatologiques de l'invention dans des concentrations allant préférentiellement de 0,01 à 20% en poids par rapport au poids total de la composition et plus préférentiellement de 0,1 à 10% en poids.

Les compositions conformes à la présente invention, sont plus particulièrement des dispersions huile-dans-eau dans lesquelles les vésicules lipidiques agissent comme agent dispersant de l'huile dans la phase continue aqueuse.

Les vésicules lipidiques selon l'invention peuvent encapsuler une phase aqueuse (coeur aqueux) ou une phase huileuse (coeur huileux). Ils sont choisis parmi ceux décrits et préparés dans la demande de brevet EP-A-0 728 459.

Les vésicules lipidiques à coeur aqueux conformes à l'invention sont des vésicules comprenant une membrane lipidique obtenue à partir de lipides amphiphiles non-ionique, de lipides amphiphiles ioniques ou de leurs mélanges.

Les vésicules lipidiques à coeur huileux conformes à l'invention sont en particulier des globules huileux en dispersion aqueuse enrobés d'une couche monolamellaire ou oligolamellaire obtenue à partir d'au moins un tensioactif lipophile, un tensioactif hydrophile et soit un lipide amphiphile ionique soit un acide gras associé à un agent basique dissous dans la phase aqueuse de la dispersion. On entend par couche "oligolamellaire" une couche comprenant de 2 à 5 lamelles lipidiques. Ces vésicules sont décrites dans les demandes de brevet français n° 94-12005 et 93-10588. Elles sont préparées selon un procédé consistant à mélanger dans une première étape, sous agitation, la phase huileuse comprenant l'agent tensioactif hydrophile, l'agent tensioactif lipophile, le lipide amphiphile ionique ou l'acide gras et la phase aqueuse contenant éventuellement l'agent basique, puis dans une deuxième étape, à soumettre le mélange obtenu à une homogénéisation basée sur le principe de la cavitation. Cette homogénéisation est obtenue soit à l'aide d'ultrasons, soit à l'aide de hautes pressions comprises entre 200 et 1500 bars, soit à l'aide d'homogénéisateurs équipés d'une tête rotor-stator.

Les lipides non-ioniques amphiphiles utilisés pour la préparation des vésicules à coeur aqueux sont choisis de préférence dans le groupe formé par :

1) les éthers de polyglycérol, linéaires ou ramifiés, de formule :

$$R^{10}O\text{-}[\text{-}C_3H_5\text{-}(OH)O\text{-}]_n\text{-}H \qquad (II)$$

dans laquelle :
$-C_3H_5(OH)O$ est représenté par les structures suivantes prises en mélange ou séparément :

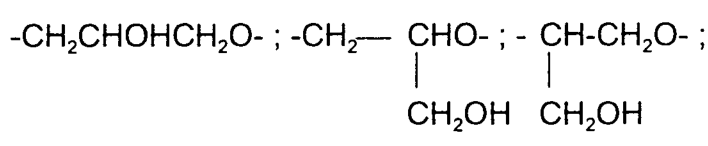

. n est une valeur statistique moyenne comprise entre 2 et 6 ;
. $R^{10}$ représente :

(a) une chaîne aliphatique, linéaire ou ramifiée, contenant de 12 à 18 atomes de carbone ;
(b) un reste $R^{11}CO$, où $R^{11}$ est un radical aliphatique, linéaire ou ramifié, en $C_{11}$-$C_{17}$ ;
(c) un reste $R^{12}$-[-$OC_2H_3(R^{13})$-]-, où :

. $R^{12}$ peut prendre la signification (a) ou (b) donnée pour $R^{10}$ ;

. $OC_2H_3(R^{13})$- est représenté par les structures suivantes, prises en mélange ou séparément:

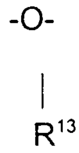

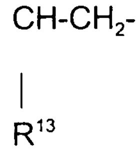

et -O-$CH_2$-CH- où $R^{13}$ prend la signification (a) donnée pour $R^{10}$ ;

(2) les alcools gras polyoxyéthylénés, les stérols polyoxyéthylénés ;
(3) les esters de polyols éventuellement polyoxyéthylénés ;
(4) les glycolipides naturels ou synthétiques ; et
(5) le stéarate de polyglycérol oxyéthyléné.

Les lipides ioniques amphiphiles utilisés selon l'invention pour la préparation des vésicules lipidiques à coeur huileux ou à coeur aqueux, sont choisis de préférence parmi les lipides anioniques neutralisés, les lipides amphotères et les dérivés alkylsúlfoniques et plus particulièrement dans le groupe formé par :

- les sels alcalins du dicétyl et du dimyristylphosphate,
- les sels alcalins du cholestérol sulfate,
- les sels alcalins du cholestérol phosphate,
- les acylglutamates mono et disodique, et en particulier les sels mono- et disodiques de l'acide N-stéaroylglutamique ;
- les sels de sodium de l'acide phosphatidique,
- les dérivés alkylsulfoniques de formule :

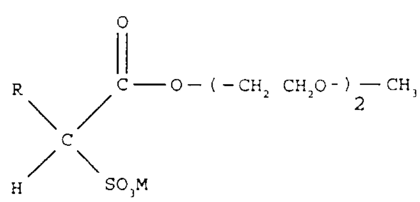

formule dans laquelle R représente les radicaux $C_{16}$ $H_{33}$ et $C_{18}$ $H_{37}$ pris en mélange ou séparément et M est un métal alcalin.

- les phosphoaminolipides ;
- les phospholipides naturels, tels que la lécithine d'oeuf ou de soja, la sphingomyéline, la phosphatidylsérine, la dipalmitoylphosphatidylcholine et les lécithines hydrogénées.

Les vésicules à coeur aqueux conformes à l'invention ont avantageusement un diamètre moyen allant de 10 à 1000 nm.

Les vésicules à coeur aqueux selon l'invention sont présents dans la composition dans des proportions allant, de préférence, de 0,5 à 15 % en poids par rapport au poids total de la composition.

Une forme particulièrement préférée de vésicules à coeur aqueux consiste en des vésicules comprenant une membrane lipidique obtenue à partir d'un mélange de stérols de soja polyoxyéthylénés et de lécithine hydrogénée.

Les agents tensioactifs lipophiles et les agents tensioactifs hydrophiles utilisés pour la préparation des vésicules à coeur huileux comportent de façon préférentelle chacun au moins une chaîne grasse saturée ayant plus de 12 atomes de carbone environ. Plus préférentiellement encore, cette chaîne grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de glycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE (comportant 2 moles d'oxyde d'éthylène) le mono et dibéhénate de glycéryle, le tétrastéarate de pentaérythritol.

L'agent tensioactif hydrophile présente de préférence un HLB compris entre environ 8 et environ 12.

On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE et le distéarate de méthylglucose polyoxyéthyléné 20 OE.

L'acide gras est selon l'invention choisi de préférence parmi les acides gras saturés en $C_{16}$-$C_{22}$. On peut citer, par exemple, l'acide palmitique, l'acide stéarique, l'acide arachidique et l'acide béhénique.

L'agent basique contenu dans la phase aqueuse de la dispersion de vésicules à coeur huileux, associé à l'acide gras, est choisi par exemple parmi la soude, la triéthanolamine, la lysine ou l'arginine.

On utilise plus particulièrement des vésicules lipidiques sous forme de dispersion aqueuse de globules huileux enrobés d'une couche obtenue à partir de distéarate de saccharose, de mono-stéarate de sorbitane oxyéthyléné à 4 moles d'oxyde d'éthylène et d'un sel disodique d'acide d'acylglutamique.

Les vésicules lipidiques à coeur huileux tels que décrits ci-dessus ont de préférence une taille moyenne inférieure à 500 nanomètres et plus particulièrement inférieure à 200 nanomètres.

Les globules huileux enrobés représentent de préférence 0,5 à 50 % en poids par rapport au poids total de la composition.

Un autre objet de l'invention consiste une composition cosmétique et/ou dermatologique contenant une dispersion aqueuse de vésicules lipidiques caractérisée par le fait qu'elle contient au moins un composé acide conférant à ladite dispersion un pH acide et au moins un poly(acide 2-acrylamido 2-méthylpropane sulfonique) réticulé et neutralisé à au moins 90%.

Les compositions conformes à l'invention comprenant des actifs acides présentent un pH de préférence inférieur à 5 et plus particulièrement allant de 2,8 à 4.8

Comme actifs acides organiques solubilisables dans la composition de l'invention, on peut citer l'acide ascorbique, l'acide kojique, l'acide citrique, l'acide caféique, l'acide salicylique et ses dérivés (par exemple acide n-octanoyl-5 ou décanoyl-5-salicylique), les $\alpha$-hydroxyacides tels que l'acide lactique, l'acide méthyllactique, l'acide glucuronique, l'acide glycolique, l'acide pyruvique, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodéca-noïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque, l'acide mandélique, l'acide benzoïque, l'acide phényl-lactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-campho sulfonique), l'acide urocanique, l'acide 2-phényl benzimidazole 5-sulphonique, l'acide $\alpha$-(oxo-2-bornylidène-3) toluène-4-sulfonique, l'acide 2-hydroxy-4-méthoxy-5-sulfonique. On peut aussi, utiliser tous les composés naturels ou synthétiques contenant de tels acides, comme les extraits végétaux et plus spécialement les extraits de fruits. On peut aussi solubiliser les dérivés xanthiques acides (caféine, théophylline), l'acide $\beta$-glycyrrhétinique, l'acide asiatique.

On utilise plus particulièrement les $\alpha$-hydroxyacides issus de fruits tels que les acides glycolique, lactique, citrique, tartrique, malique, mandélique ou leurs mélanges.

Les composés acides sont présents dans les compositions de l'invention dans des proportions allant, de préférence, de 0,1 à 10% en poids par rapport au poids total de la composition et plus préférentiellement de 0,2 à 5% en poids et plus particulièrement de 0,5 à 4% en poids.

Le composé acide peut être présent dans la phase aqueuse de la dispersion de vésicules à coeur aqueux ou huileux ou bien dans la phase aqueuse interne des vésicules à coeur aqueux.

Lorsque la composition est sous forme de dispersion aqueuse de vésicules à coeur huileux ou aqueux, le composé acide peut être introduit dans la phase aqueuse externe sous agitation, dans la dispersion de vésicules déjà formée.

Une forme particulière de l'invention consiste en une composition comprenant une dispersion aqueuse de vésicules à coeur huileux en mélange avec une dispersion de vésicules à coeur aqueux encapsulant le composé acide , le composé acide est dans ce cas introduit dans la dispersion de vésicules à coeur huileux déjà formée par le biais d'une dispersion de vésicules à coeur aqueux encapsulant le composé acide hydrosoluble.

La phase aqueuse de la dispersion de la composition selon l'invention peut également contenir un liquide non miscible dans l'eau dispersé par les vésicules lipidiques.

Le liquide non miscible à l'eau, qui peut être présent sous forme de dispersion dans la phase aqueuse de dispersion, peut notamment être choisi dans le groupe formé par :

- les huiles animales ou végétales formées par des esters d'acide gras et de polyols, en particulier les triglycérides liquides, par exemple les huiles de tournesol, de maïs, de soja, de courge, de pépins de raisin, de jojoba, de sésame, de noisette, les huiles de poisson, le tri-caprocaprylate de glycérol, ou les huiles végétales ou animales de formule $R_8COOR_9$, formule dans laquelle $R_8$ représente le reste d'un acide gras supérieur comportant de 7 à 19 atomes de carbone et $R_9$ représente une chaîne hydrocarbonée ramifiée contenant de 3 à 20 atomes de carbone, par exemple l'huile de Purcellin ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- des hydrocarbures, tels que l'hexadécane et l'huile de paraffine ;
- des carbures halogénés, notamment des fluorocarbures tels que des fluoroamines par exemple la perfluorotributylamine, des hydrocarbures fluorés, par exemple le perfluorodécahydronaphtalène, des fluoroesters et des fluoroéthers ;
- des silicones, par exemple les polysiloxanes, les polydiméthylsiloxanes et les fluorosilicones ;
- des esters d'acide minéral et d'un alcool ; et
- des éthers et des polyéthers.

La phase aqueuse de dispersion peut aussi également contenir des actifs cosmétiques et/ou dermopharmaceutiques, hydrosolubles. Le liquide non-miscible à l'eau peut éventuellement contenir un actif liposoluble.

La phase aqueuse de dispersion peut aussi contenir des adjuvants n'ayant ni activité cosmétique ni activité dermopharmaceutique propre, mais utilisés pour la formulation de la dispersion sous forme de lotion, crème ou sérum. Ces adjuvants sont, en particulier, pris dans le groupe formé par les conservateurs, les colorants, les pigments, les opacifiants, les parfums, les filtres solaires et les poudres à usage cosmétique.

La phase aqueuse de la dispersion peut contenir des pigments, des nanopigments, des nanotitanes enrobés ou non, des colorants solubles. Des poudres matifiantes peuvent être également ajoutées. On peut citer, par exemple, les poudres d'amidon, de Nylon, les microsphères de silice, les plaquettes de mica recouvertes de microsphères de silice.

Un autre objet de l'invention porte sur l'utilisation des compositions définies cidessus comme base de produits de soin ou de maquillage (crème teintée, fond de teint) pour le visage et/ou le corps.

Ces produits peuvent se présenter sous la forme de dispersions plus ou moins épaissies, de gels, de crèmes, de laits ou de sérums.

Enfin l'invention porte sur l'utilisation de la composition telle que définie précédemment dans et pour la préparation de produits cosmétiques destinés au traitement des rides et/ou ridules de la peau ou des comédons ainsi qu'un procédé de traitement cosmétique des rides et/ou des ridules de la peau ou des comédons, consistant à appliquer sur la peau une quantité efficace de la composition de l'invention.

D'autres caractéristiques et avantages de l'invention ressortiront mieux des exemples qui suivent, donnés à titre illustratif et non limitatif.

## EXEMPLE DE PREPARATION A

Dans un ballon de 5 litres muni d'un agitateur, d'un réfrigérant à reflux, d'un thermomètre et d'un dispositif de conduite pour l'azote et pour l'ammoniaque, on introduit 2006,2 g de tertio-butanol puis 340,0 g d'acide 2-acrylamido 2-méthyl-propane sulfonique que l'on disperse dans la solution sous forte agitation. Après 30 minutes, on ajoute l'ammoniaque par le conduit supérieur du ballon et on maintient le mélange réactionnel pendant 30 minutes à température ambiante jusqu'à l'obtention d'un pH de l'ordre de 6-6,5. On introduit ensuite 32,0 g d'une solution de triméthylolpropane triacrylate à 25% dans le tertio-butanol et on chauffe jusqu'à 60°C tandis que le milieu réactionnel est simultanément rendu inerte par apport de l'azote dans le ballon. Une fois cette température atteinte, on ajoute du dilauroylperoxyde. La réaction se déclenche aussitôt, ce qui se traduit par une montée de température et par une précipitation du polymérisat. 15 minutes après le début de la polymérisation, on introduit un courant d'azote. 30 minutes après l'ajout de l'amorceur, la température du milieu réactionnel atteint un maximum de 65-70°C. 30 minutes après avoir atteint cette température, on chauffe au reflux et on maintient dans ces conditions pendant 2 heures. On observe au cours de la réaction la formation d'une pâte épaisse. On refroidit jusqu'à la température ambiante et on filtre le produit obtenu. La pâte récupérée est ensuite séchée sous vide à 60-70°C pendant 24 heures. On obtient 391 g de poly(acide 2-acrylamido 2-méthylpropane sulfonique) réticulé et neutralisé, ayant viscosité mesurée au viscosimètre BROOKFIELD, mobile 4, à une vitesse de rotation de 100 tours/minutes dans une solution d'eau à 2 % et à 25 °C allant de 13 000 cps à 35.000 cps. La viscosité du polymère sera choisie et contrôlée selon des moyens classiques en fonction de l'application cosmétique envisagée.

Le rayon hydrodynamique du polymère obtenu dans une solution aqueuse déterminé par diffusion de la lumière dynamique est de 440 nm.

**EXEMPLE 1 : Fond de teint (dispersion de vésicules à coeur aqueux de pH 3,3)**

Phase $A_1$ :

| | |
|---|---|
| Stérols de soja oxyéthylénés à 5 OE non stabilisés vendus sous le nom GENEROL 122E5 | 1,6 g |
| Lécithine hydrogénée vendue sous le nom LECINOL S10 | 2,4 g |

Phase $A_2$ :

| | |
|---|---|
| Eau déminéralisée stérile | 15 g |

Phase $A_3$ :

| | |
|---|---|
| Eau déminéralisée stérile | 14 g |
| Conservateur | 0,2 g |
| Glycérine | 3 g |
| Propylèneglycol | 3 g |

Phase $B_1$ :

| | |
|---|---|
| Huile de palme | 6,5 g |
| Huile d'amande d'abricots désodorisée non stabilisée | 9,5 g |
| Conservateurs | 0,19 g |

Phase $B_2$ :

| | |
|---|---|
| Cyclopentadiméthylsi-loxane | 10 g |

Phase $B_2$ :

| | |
|---|---|
| Acétate de vitamine E | 0,5 g |
| Filtre solaire | 1 g |

Phase C :

| | |
|---|---|
| Pigments | 7 g |

Phase D :

| | |
|---|---|
| Eau déminéralisée stérile | 1 g |
| Conservateur | 0,3 g |

Phase E :

| | |
|---|---|
| Eau déminéralisée stérile | 18,84 g |
| Poly(acide 2-acrylamido 2-méthylpropane sulfonique) MA réticulé et neutralisé par de l'ammoniaque, préparé selon le procédé de l'exemple de préparation A | 0,9 g |

Phase F :

| | |
|---|---|
| Mélange d'alphahydroxyacides issus de fruits (acide lactique/glycolique/citrique 30/15/4) dans l'eau (pH = 2). | 1 g |

Phase G :

| | |
|---|---|
| Poudre d'amidon | 3 g |
| Eau déminéralisée stérile qsp | 100 g |

**Mode opératoire**

I - PREPARATION DE LA PHASE VESICULAIRE A :

*Préparation de $A_1$:*

On introduit l'eau de la partie $A_1$ à 85 ° C. On ajoute les stérols de soja oxyéthylénés (GENEROL 122 E5) sous agitation. On obtient une huile jaune et visqueuse. On ajoute la lécithine hydrogénée, sous agitation pendant 15 min. On obtient une pâte beige friable. On maintient la température à 85 ° C, sous agitation.

*Préparation de $A_3$ :*

On introduit l'eau de $A_3$ à 85° C.
On ajoute, le propylèneglycol, la glycérine et le conservateur, sous agitation Moritz à 300 t/min.

*Mise en oeuvre de la phase vésiculaire A :*

Sur $A_1$, on ajoute $A_2$ (eau) à 85° C en 3 fois sous agitation, pendant 15 minutes. On laisse le mélange $A_1$ + $A_2$ gonfler pendant 1 heure.
On introduit, par le haut de la cuve, $A_3$ à 85° C, sous agitation. On met sous agitation pendant 10 min. On refroidit à 55° C sous agitation.

*Passage homogénéisation haute pression.*

A 55° C, on effectue 3 passages à l'homogénéisateur haute pression (500 bars).
Après le troisième passage, on transfère le produit et on refroidit à 40° C sous agitation.

II - PREPARATION DE $B_1$ + $B_2$ + $B_3$

On introduit l'huile de palme, l'huile d'amandes d'abricots, le conservateur. On chauffe à 80° C, sous agitation Morts 500 t/min, jusqu'à homogénéisation complète. On refroidit à 65 ° C. On introduit la silicone volatile. On ajoute l'acétate de vitamine E et le filtre solaire, sous agitation Moritz 700 t/min. On refroidit à 40° C.

III - PREPARATION DE LA DISPERSION VESICULAIRE + HUILES

On introduit la partie B dans la partie A. On met sous agitation, pendant 10 min. On refroidit à 25° C sous agitation.
On effectue 6 passage à l'homogénéisateur haute pression (500 bars).

IV - DISPERSION DES PIGMENTS

On transfère une partie de produit, on y ajoute les pigments (phase C). On met sous agitation pendant 1 heure. On vérifie la dispersion de C au microscope (il ne doit pas y avoir d'amas de pigments supérieur à 30 microns).
On transfère le reste de produit sous agitation. On maintient la température à 30° C.

V - PREPARATION DE D

On solubilise le conservateur dans l'eau à température ambiante.

VI - PREPARATION DE E

On disperse le polymère épaississant dans l'eau chaude à température 80°C, à la défloculeuse.

VII - AJOUT DES PHASES D, E, F, G

On ajoute D et on homogénéise sous agitation pendant 5 minutes.
On ajoute E et on homogénéise pendant 5 min sous agitation. On vérifie la bonne dispersion du gel au microscope. On ajoute le mélange d'alphahydroxyacides (phase F) puis l'amidon (G) sous agitation pendant 5 min. On refroidit à 25° C sous agitation puis on arrête l'agitation. On effectue un prélèvement pour contrôle.
La composition obtenue est stable à température ambiante après 5 mois et demi de stockage. Elle est également stable après 2 mois de stockage en étuve à 45° C.

**EXEMPLE 2 : CREME TEINTEE (dispersion de vésicules à coeur aqueux)**

La composition est préparée selon le même mode opératoire de l'exemple 1et comprend les ingrédients suivants :

| | |
|---|---|
| Stérols de soja oxyéthylénés à 5 OE vendus sous le nom GENEROL 122 E5 | 1,6 g |
| Lécithine hydrogénée LECINOL S10 | 2,4 g |
| Mélange de tocophérols naturels dans huile de soja(50/50) | 0,2 g |
| Mélange d'huiles raffinées de maïs/son de riz/sésame/germes de blé(29/40/20/10) | 5 g |
| Huile d'amandes d'abricot désodorisée non stabilisée | 7 g |
| Propylène glycol | 3 g |
| Filtre solaire | 2 g |
| Conservateurs | 0,35 g |
| Cyclohexadiméthylsiloxane | 10 g |
| Vitamines | 2,8 g |
| Pigments | 5 g |
| Mélange d'alphahydroxyacides issus de fruits (acide lactique/glycolique/citrique 30/15/4) dans l'eau | 1 g |
| Poly(acide 2-acrylamido 2-méthylpropane sulfonique) MA réticulé et neutralisé par de l'ammoniaque, préparé selon le procédé de l'exemple de préparation A | 0,9 g |
| Eau déminéralisée | 59,75 g |

La composition obtenue est stable à température ambiante après 5 mois et demi de stockage. Elle est également stable après 2 mois de stockage à 45° C.

**EXEMPLE 3 : Fond de teint (dispersion de vésicules à centre huileux de pH 4,7)**

Phase $A_1$ :

| | |
|---|---|
| Distéarate de saccharose | 2,4 g |
| Stéarate de sorbitane oxyéthylèné à 4 moles d'oxyde d'éthylène | 1,6 g |
| Sel disodique de l'acide N-stéaroyl glutamique | 1,2 g |
| Mélanges d'huiles de tournesol, rosier, muscat, pépins de cassis (31/60/5,95/3) | 5 g |
| Isoparaffine hydrogénée | 9 g |
| Polydiméthylsiloxane de viscosité 5 cst | 9 g |
| Vitamine E | 0,09 g |
| Filtre solaire | 1 g |

Phase $A_2$ :

| | |
|---|---|
| Eau déminéralisée stérile | 35 g |
| EDTA | 0,05 g |
| Glycérine | 4 g |

Phase $B_1$ :

| | |
|---|---|
| Eau déminéralisée stérile | 11,85 g |
| Silicate de sodium et magnésium | 0,3 g |

Phase $B_2$ :

| | |
|---|---|
| Pigments | 9 g |

Phase C :

| | |
|---|---|
| Eau déminéralisée stérile | 1 g |
| Conservateur | 0,3 g |

Phase D :

| | |
|---|---|
| Polyéthylèneglycol (80 OE) | 5 g |

Phase E :

| | |
|---|---|
| Poudre matifiante | 2 g |

Phase F :

| | |
|---|---|
| Poly(acide 2-acrylamido 2-méthylpropane sulfonique) MA réticulé et neutralisé par de l'ammoniaque, préparé selon le procédé de l'exemple de préparation A | 0,4 g |

Phase G :

| | |
|---|---|
| Acide lactique dans l'eau | 1 g |
| Eau déminéralisée stérile qsp | 100 g |

**Mode opératoire** :

I - FABRICATION DE LA PHASE VESICULAIRE A

On fait fondre la phase A1 à 85-90° C.

On homogénéise.

En parallèle, on prépare la phase $A_2$ (dissolution de l'EDTA) et on l'amène à 60° C.

On verse la phase $A_2$ sur la phase $A_1$, rapidement et sous agitation vive. On homogénéise à 60 °C pendant 10 min. On passe l'ensemble à l'homogénéisation haute pression, sous 500 bars. 2 passages au minimum sont nécessaires. On doit obtenir un diamètre de 200 nm pour une polydispersité inférieure à 1.

On refroidit à 30° C et on désaère.

II - PREPARATION DE LA DISPERSION PIGMENTAIRE

En parallèle, à l'aide d'un *homogène-agitateur*, on prépare le gel de silicate de magnésium et de sodium.

On disperse dans le gel les pigments (phase $B_2$). On refroidit jusqu'à 35° C en 15 min. On introduit la phase C (conservateur).

On continue la dispersion en 1 heure et demie au total.

III - DISPERSION DE LA DISPERSION VESICULAIRE DANS LA DISPERSION PIGMENTAIRE

On introduit en plusieurs étapes, la phase vésiculaire à 30° C sur la dispersion pigmentaire sous agitation turbine et pâles à grande vitesse.

On laisse turbiner pendant 1 heure. On ajoute par gravité la phase D. On homogénéise pendant 5 minutes.

On ajoute la phase E (poudre) et on homogénéise pendant 2 minutes. On ajoute la phase F (gélifiant) et on homogénéise pendant 10 minutes. On désaère au maximum.

IV - INTRODUCTION DES ALPHA-HYDROXYACIDES

A la formule ainsi obtenue sous forme gélifiée, on ajoute, sous agitation pâles vitesse rapide, la phase G. On homogénéise pendant 5 minutes.

La composition obtenue est stable à température ambiante.

**EXEMPLE 4 : Fond de teint (dispersion de vésicules à centre huileux)**

La composition est préparée selon le même mode opératoire de l'exemple 3 et comprend les ingrédients suivants :

| | |
|---|---|
| Distéarate de saccharose | 2,4 g |
| Stéarate de sorbitane oxyéthyléné à 4 moles d'oxyde d'éthylène | 1,6 g |
| Sel disodique de l'acide N-stéaroyl glutamique | 1,2 g |
| Mélange d'huiles raffinées de maïs/son de riz/sésame/germes de blé(29/40/20/10) | 5 g |
| Huile d'amandes d'abricot désodorisée | 2 g |
| Huile d'amandes douces non stablisée | 2 g |
| Isoparaffine hydrogénée | 9 g |
| Polydiméthylsiloxane de viscosité 5 cst | 9 g |
| Cyclohexadiméthylsiloxane de viscosité 8 cst | 2 g |
| Vitamine E | 0,5 g |
| Filtre solaire | 1 g |
| EDTA | 0,02 g |
| Glycérine | 5 g |
| Silicate de sodium et magnésium | 0,3 g |
| Pigments | 7 g |
| Eau déminéralisée stérile | 1 g |
| Conservateurs | 0,75 g |

(suite)

| | |
|---|---|
| Poudre d'amidon | 2 g |
| Poly(acide 2-acrylamido 2-méthylpropane sulfonique) MA réticulé et neutralisé par de l'ammoniaque, préparé selon le procédé de l'exemple de préparation A | 0,25 g |
| Eau déminéralisée stérile | 54,18 g |

**EXEMPLE 5: Fond de teint (dispersion de vésicules à coeur huileux et de vésicules à coeur aqueux encapsulant les α-hydroxyacides pH 4,7)**

Phase $A_1$ :

| | |
|---|---|
| Distéarate de saccharose | 1,8 g |
| Stéarate de sorbitane oxyéthyléné à 4 moles d'oxyde d'éthylène | 1,6 g |
| Sel disodique de l'acide N-stéaroyl glutamique | 0,9 g |
| Mélange d'huiles de tournesol, rosier, muscat, pépins de cassis (31/60/5,95/3) stabilisé | 3,70 g |
| Isoparaffine hydrogénée (8 OE) | 6,70 g |
| Polydiméthylsiloxane 5 cst | 6,70 g |
| Vitamine E | 0,06 g |
| Filtre solaire | 0,70 g |

Phase $A_2$ :

| | |
|---|---|
| Eau déminéralisée stérile | 26,12 g |
| EDTA, disodique | 0,03 g |
| Glycérine | 2,98 g |

Phase $B_1$ :

| | |
|---|---|
| Eau déminéralisée stérile | 7,24 g |
| Silicate de sodium et magnésium | 0,22 g |

Phase $B_2$:

| | |
|---|---|
| Pigments | 6,72 g |

Phase C :

| | |
|---|---|
| Eau déminéralisée | 0,74 g |
| Conservateur | 0,22 g |

Phase D :

| | |
|---|---|
| Polyéthylène glycol (8 OE) | 3,73 g |

Phase E :

| | |
|---|---|
| Poudre matifiante | 1,49 g |

Phase F :

| | |
|---|---|
| Poly(acide 2-acrylamido 2-méthylpropane sulfonique) MA réticulé et neutralisé par de l'ammoniaque, préparé selon le procédé de l'exemple de préparation A | 0,3 g |

Phase $G_1$ :

| | |
|---|---|
| GENEROL 122 E5 | 1,20 g |
| LECINOL S10 | 1,8 g |

(suite)

| | |
|---|---|
| Eau déminéralisée stérile | 11,20 g |

Phase $G_2$ :

| | |
|---|---|
| Eau déminéralisée stérile | 10,45 g |
| Conservateur | 0,07 g |
| Propylène glycol | 2,24 g |
| Mélange d'acides lactique/glycolique/citrique (30/15/4) dans l'eau pH = 2 | 0,75 g |
| Eau déminéralisée stérile qsp | 100 g |

**Mode opératoire**

La dispersion de vésicules à coeur huileux est préparée à partir des phases $A_1$, $A_2$, $B_1$, $B_2$, C, D, E et F selon les mêmes étapes du procédé de l'exemple 3 précédant l'introduction des $\alpha$-hydroxyacides.

On réalise une phase vésiculaire à centre aqueux (phase $G_1$) dans laquelle on ajoute lors de la seconde hydratation la phase $G_2$ contenant les $\alpha$-hydroxyacides.

Cette phase vésiculaire est alors considérée comme actif et est ajoutée, sous agitation pâles vitesse rapide à la dispersion de vésicules à coeur huileux déjà formée.

La composition obtenue est stable à température ambiante.

**EXEMPLE 6 : (Comparatif)**

La Demanderesse a réalisé les trois compositions suivantes :

- Composition A : (invention)

Phase $A_1$ :

| | |
|---|---|
| Stérols de soja oxyéthylénés à 5 OE non stabilisés vendus sous le nom GENEROL 122E5 | 1,6 g |
| Lécithine hydrogénée vendue sous le nom LECINOL S10 | 2,4 g |

Phase $A_2$ :

| | |
|---|---|
| Eau déminéralisée stérile | 15 g |

Phase $A_3$ :

| | |
|---|---|
| Eau déminéralisée stérile | 14 g |
| Conservateur | 0,2 g |
| Glycérine | 3 g |
| Propylèneglycol | 3 g |

Phase $B_1$ :

| | |
|---|---|
| Huile d'amande d'abricots désodorisée non stabilisée | 12 g |
| Conservateurs | 0,15 g |

Phase $B_3$ :

| | |
|---|---|
| Cyclohexadiméthylsiloxane | 12 g |

Phase $B_3$ :

| | |
|---|---|
| Filtre solaire | 2 g |

Phase C :

| | |
|---|---|
| Eau déminéralisée stérile | 1 g |
| Conservateur | 0,3 g |

Phase D :

| | |
|---|---|
| Eau déminéralisée stérile | 35.28 g |
| Poly(acide 2-acrylamido 2-méthylpropane sulfonique) MA réticulé et neutralisé par de l'ammoniaque, préparé selon le procédé de l'exemple de préparation A | 0,72 g |

Phase E :

| | |
|---|---|
| Eau déminéralisée stérile qsp | 100 g |

- Composition B : (comparatif)

Phase $A_1$ :

| | |
|---|---|
| Stérols de soja oxyéthylénés à 5 OE non stabilisés vendus sous le nom GENEROL 122E5 | 1,6 g |
| Lécithine hydrogénée vendue sous le nom LECINOL S10 | 2,4 g |

Phase $A_2$ :

| | |
|---|---|
| Eau déminéralisée stérile | 15 g |

Phase $A_2$ :

| | |
|---|---|
| Eau déminéralisée stérile | 14 g |
| Conservateur | 0,2 g |
| Glycérine | 3 g |
| Propylèneglycol | 3 g |

Phase $B_1$ :

| | |
|---|---|
| Huile d'amande d'abricots désodorisée non stabilisée | 12 g |
| Conservateurs | 0,15 g |

Phase $B_2$ :

| | |
|---|---|
| Cyclohexadiméthylsiloxane | 12 g |

Phase $B_3$ :

| | |
|---|---|
| Filtre solaire | 2 g |

Phase C :

| | |
|---|---|
| Eau déminéralisée stérile | 1 g |
| Conservateur | 0,3 g |

Phase D :

| | |
|---|---|
| Mélange de polyacrylamide, isoparaffine, alcool laurylique oxyethyléné dans l'eau (40/34.5/5.5/20) | 1.8 g soit 0.72g MA |

Phase E :

| | |
|---|---|
| Eau déminéralisée stérile qsp | 100 g |

- Composition C : (comparatif)

Phase $A_1$ :

| | |
|---|---|
| Stérols de soja oxyéthylénés à 5 OE non stabilisés vendus sous le nom GENEROL 122E5 | 1,6 g |
| Lécithine hydrogénée vendue sous le nom LECINOL S10 | 2,4 g |

Phase $A_2$ :

| | |
|---|---|
| Eau déminéralisée stérile | 15 g |

Phase $A_3$ :

| | |
|---|---|
| Eau déminéralisée stérile | 14 g |
| Conservateur | 0,2 g |
| Glycérine | 3 g |
| Propylèneglycol | 3 g |

Phase $B_1$ :

| | |
|---|---|
| Huile d'amande d'abricots désodorisée non stabilisée | 12 g |
| Conservateurs | 0,15 g |

Phase $B_2$ :

| | |
|---|---|
| Cyclohexadiméthylsiloxane | 12 g |

Phase $B_2$ :

| | |
|---|---|
| Filtre solaire | 2 g |

Phase C :

| | |
|---|---|
| Eau déminéralisée stérile | 1 g |
| Conservateur | 0,3 g |

**Mode opératoire**

I - PREPARATION DE LA PHASE VESICULAIRE A :

*Préparation de $A_1$:*

On introduit l'eau de la partie $A_1$ à 85 ° C. On ajoute les stérols de soja oxyéthylénés (GENEROL 122 E5) sous agitation. On obtient une huile jaune et visqueuse.
On ajoute la lécithine hydrogénée, sous agitation pendant 15 min. On obtient une pâte beige friable. On maintient la température à 85 ° C, sous agitation.

*Préparation de $A_3$ :*

On introduit l'eau de $A_3$ à 85° C.
On ajoute, le propylèneglycol, la glycérine et le conservateur, sous agitation Moritz à 300 t/min.

*Mise en oeuvre de la phase vésiculaire A* :

Sur $A_1$, on ajoute $A_2$ (eau) à 85° C en 3 fois sous agitation, pendant 15 minutes. On laisse le mélange $A_1 + A_2$ gonfler pendant 1 heure.
On introduit, par le haut de la cuve, $A_3$ à 85° C, sous agitation. On met sous agitation pendant 10 min. On refroidit à 55° C sous agitation.

*Passage homogénéisation haute pression.*

A 55° C, on effectue 3 passages à l'homogénéisateur haute pression (500 bars).
Après le troisième passage, on transfère le produit et on refroidit à 40° C sous agitation.

II - PREPARATION DE $B_1 + B_2 + B_3$

On introduit l'huile d'amandes d'abricots, le conservateur. On chauffe à 80° C, sous agitation Moritz 500 t/min, jusqu'à homogénéisation complète. On refroidit à 65 ° C. On introduit la silicone volatile. On ajoute le filtre solaire, sous agitation Moritz 700 t/min. On refroidit à 40° C.

III - PREPARATION DE LA DISPERSION VESICULAIRE + HUILES

On introduit la partie B dans la partie A. On met sous agitation, pendant 10 min. On refroidit à 25° C sous agitation.
On éffectue 6 passage à l'homogénéisateur haute pression (500 bars).

IV - PREPARATION DE C

On solubilise le conservateur dans l'eau à température ambiante.

V - PREPARATION DE D DANS LA COMPOSITION A SELON L'INVENTION

On disperse le polymère épaississant dans l'eau chaude à température 80°C, à la défloculeuse.

VI - AJOUT DES PHASES D, E

On ajoute D et on homogénéise sous agitation pendant 5 minutes. On vérifie la bonne dispersion du gel au microscope.
On ajoute E et on homogénéise pendant 5 min sous agitation. On effectue un prélèvement pour contrôle.
Ces trois compositions ont été conservées 2 mois à 45 °C. Pour chacune de ces compositions, un échantillon a été congelé à -200 °C dans de l'azote pâteux puis une cryofracture a été effectuée sur chacun d'eux à l'aide d'un cryofract de type REICHERT-JUNG à -150 °C sous vide. Après métallisation au platine et au carbone et montage des empreintes sur grilles de nickel, les échantillons ont été observés au microscope électronique à transmission de type PHILIPS CM120 à des grossissements allant de 8000 à 75 000.
Les résultats observés sont rassemblés dans le tableau ci-dessous.

| Composition | Diamètre moyen des particules observées en nm |
|---|---|
| - Composition A | 540 ± 670 |
| - Composition B | 1580 ± 500 |
| - Composition C | 2000 ± 660 |

Il ressort de ces résultats que la dispersion obtenue selon l'invention est plus fine que celles obtenues dans les compositions de l'art antérieur.

**Revendications**

1. Composition cosmétique et/ou dermatologique comprenant une dispersion aqueuse de vésicules lipidiques, caractérisée par le fait qu'elle contient au moins un poly(acide 2-acrylamido 2-méthylpropane sulfonique) réticulé neutralisé à au moins 90 %.

2. Composition selon la revendication 1, caractérisée par le fait que le poly(acide 2-acrylamido 2-méthylpropane sulfonique) réticulé et neutralisé à au moins 90% comprend, distribués de façon aléatoire :

   a) de 90 à 99,9% en poids de motifs de formule générale (1) suivante :

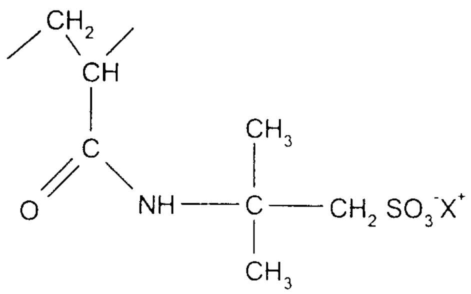 (1)

dans laquelle $X^+$ désigne un cation ou un mélange de cations, au plus 10% mol des cations $X^+$ pouvant être des protons $H^+$ ;

b) de 0,01 à 10% en poids de motifs réticulants provenant d'au moins un monomère ayant au moins deux doubles-liaisons oléfiniques ; les proportions en poids étant définis par rapport au poids total du polymère.

3. Composition selon la revendication 2, caractérisée par le fait que le poly(acide 2-acrylamido 2-méthylpropane sulfonique) réticulé comporte un nombre de motifs de formule (1) dans une quantité suffisamment élevée pour obtenir un volume hydrodynamique du polymére en solution d'eau ayant un rayon allant de 10 à 500 nm, de distribution homogène et unimodale.

4. Composition selon l'une quelconque des revendications 2 ou 3, caractérisée par le fait que le poly(acide 2-acrylamido 2-méthylpropane sulfonique) réticulé comporte de 98 à 99,5 % en poids de motifs de formule (1) et de 0,2 à 2 % en poids de motifs réticulants.

5. Composition selon l'une quelconque des revendications 2 à 4, caractérisée par le fait que dans la formule (1) le cation $X^+$ est $NH_4^+$.

6. Composition selon l'une quelconque des revendications 2 à 5, caractérisée par le fait que les monomères réticulants répondent à la formule générale (2) suivante:

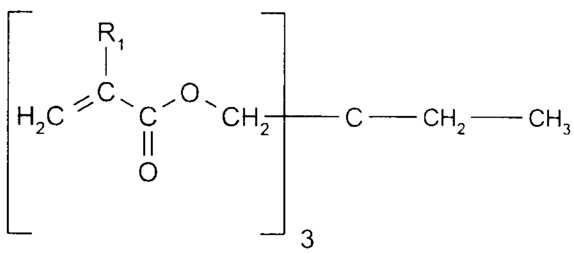 (2)

dans laquelle $R_1$ désigne un atome d'hydrogène ou un alkyle en $C_1$-$C_4$.

7. Composition selon l'une quelconque des revendications 1 à 6, caractérisée par le fait que le poly(acide 2-acrylamido 2-méthylpropane sulfonique) est réticulé par le triméthylol propane triacrylate.

8. Composition selon l'une quelconque des revendications 1 à 7, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minutes, dans une solution d'eau à 2 % et à 25 °C, supérieure ou égale à 1000 cps.

9. Composition selon l'une quelconque des revendications 1 à 8, caractérisée par le fait que le poly(acide 2-acrylamido 2-méthylpropane sulfonique) réticulé présente une viscosité mesurée au viscosimètre BROOKFIELD, mobile 4, à une vitesse de rotation de 100 tours/minutes, dans une solution d'eau à 2% et à 25°C allant de 5000 à 40000 cps et plus particulièrement de 6500 à 35000 cps.

10. Composition selon l'une quelconque des revendications 1 à 9, caractérisée par le fait que le poly(acide 2-acrylamido 2-méthylpropane sulfonique) réticulé est présent dans des concentrations allant préférentiellement de 0,01 à 20% en poids par rapport au poids total de la composition et plus préférentiellement de.0,1 à 10% en poids.

11. Composition selon l'une quelconque des revendications 1 à 10, caractérisée par le fait qu'elle est une dispersion huile-dans-eau et que les vésicules lipidiques servent de dispersant de l'huile dans la phase aqueuse de la dispersion.

12. Composition selon l'une quelconque des revendications 1 à 11, caractérisée par le fait que les vésicules lipidiques sont des vésicules à coeur huileux ou aqueux.

13. Composition selon la revendication 12, caractérisée par le fait que les vésicules à coeur aqueux comprennent une membrane lipidique obtenue à partir de stérols de soja oxyéthylénés et de lécithine hydrogénée.

14. Composition selon l'une quelconque des revendications 12 ou 13, caractérisée par le fait que les vésicules à coeur huileux comprennent une membrane lipidique obtenue à partir de distéarate de saccharose, de monostéarate de sorbitane oxyéthyléné à 4 moles d'oxyde d'éthylène et d'un sel disodique d'acide d'acylglutamique.

15. Composition selon l'une quelconque des revendications 12 à 14, caractérisée par le fait que les vésicules à coeur aqueux sont présents dans des proportions allant de 0,5 à 15 % en poids par rapport au poids total de la composition.

16. Composition selon l'une quelconque des revendications 12 à 15, caractérisée par le fait que les vésicules à coeur huileux sont présents dans des proportions allant de 0,5 à 50 % en poids par rapport au poids total de la composition.

17. Composition selon l'une quelconque des revendications 1 à 16, comprenant en plus au moins un composé acide cosmétiquement ou dermatologiquement actif.

18. Composition selon la revendication 17, caractérisée par le fait que le composé acide est présent dans la phase aqueuse externe de la dispersion.

19. Composition selon la revendication 17, caractérisée par le fait que le composé acide est encapsulé dans la phase aqueuse interne des vésicules à coeur aqueux.

20. Composition selon la revendication 17, caractérisée par le fait qu'elle comprend une dispersion de vésicules à coeur huileux en mélange avec une dispersion de vésicules à coeur aqueux encapsulant le composé acide.

21. Composition selon l'une quelconque des revendications 17 à 20, caractérisée par le fait que le pH est inférieur à 5 et de préférence varie de 2,8 à 4,8.

22. Composition selon l'une quelconque des revendications 17 à 21, caractérisée par le fait que le composé acide est présent dans des proportions allant de 0,1 à 10% en poids par rapport au poids total de la composition.

23. Composition selon l'une quelconque des revendications 17 à 22, caractérisée par le fait que le composé acide est choisi dans le groupe constitué par l'acide ascorbique, l'acide kojique, l'acide citrique, l'acide caféique, l'acide salicylique et ses dérivés, l'acide lactique, l'acide méthyllactique, l'acide glucuronique, l'acide glycolique, l'acide pyruvique, l'acide 2-hydroxy-butanoïque, l'acide 2-hydroxy-pentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxy-décanoïque, l'acide 2-hydroxyundécanoïque, l'acide 2-hydroxydodécanoïque, l'acide 2-hydroxy-tétradécanoïque, l'acide 2-hydroxy-hexadécanoïque, l'acide 2-hydroxy-octad-écanoïque, l'acide 2-hydroxytétra-cosanoïque, l'acide 2-hydroxyeïcosanoïque, l'acide mandélique, l'acide benzoïque, l'acide phényllactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique), l'acide urocanique, l'acide 2-phényl benzimidazole 5-sulphonique, l'acide α-(oxo-2-bornylidène-3) toluène-4-sulfonique, l'acide 2-hydroxy-4-méthoxy-5-sulfonique, les dérivés xanthiques acides (caféine, théophylline), l'acide β-glycyrrhétinique, l'acide asiatique.

24. Composition selon la revendication 23, caractérisée par le fait que le composé acide est choisi parmi les α-hydroxyacides issus de fruits en particulier les acides glycolique, lactique, citrique, tartrique, malique, mandélique ou leurs mélanges.

**25.** Utilisation d'une composition selon l'une quelconque des revendications 1 à 24 comme base de produits pour le soin ou le maquillage du corps et/ou du visage.

**26.** Utilisation d'une composition selon l'une quelconque des revendications 1 à 24 pour dans et pour la préparation de produits cosmétiques destinés au traitement des rides et/ou ridules de la peau ou des comédons dus à l'acné.

**27.** Procédé cosmétique de traitement des ridules et/ou des rides de la peau ou des comédons caractérisée par le fait qu'on applique sur la peau une quantité efficace d'une composition selon l'une quelconque des revendications 1 à 24.

**28.** Composition selon l'une quelconque des revendications 1 à 24 caractérisée en ce que le poly(acide 2-acrylamido 2-méthylpropane sulfonique) réticulé neutralisé à au moins 90 % est un homopolymère.

Office européen des brevets

# RAPPORT DE RECHERCHE EUROPEENNE

Numéro de la demande
EP 98 40 1443

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | EP 0 642 781 A (L'OREAL) 15 mars 1995<br>* le document en entier *<br>--- | 1 | A61K7/00<br>A61K7/48 |
| X | FR 2 698 004 A (L'OREAL) 20 mai 1994<br>* revendication 1 *<br>--- | 1 | |
| A | K. ISHIHARA ET AL.: "SPECIFIC INTERACTION BETWEEN WATER-SOLUBLE PHOSPHOLIPID POLYMER AND LIPOSOME"<br>JOURNAL OF POLYMER SCIENCE, PART A,<br>vol. 29, 1991, pages 831-835, XP002060310<br>----- | 1 | |

| DOMAINES TECHNIQUES RECHERCHES (Int.Cl.6) |
|---|
| A61K |

Le présent rapport a été établi pour toutes les revendications

| Lieu de la recherche | Date d'achèvement de la recherche | Examinateur |
|---|---|---|
| LA HAYE | 12 février 1999 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

1

EPO FORM 1503 03.82 (P04C02)

## ANNEXE AU RAPPORT DE RECHERCHE EUROPEENNE RELATIF A LA DEMANDE DE BREVET EUROPEEN NO. EP 98 40 1443

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-02-1999

| Document brevet cité au rapport de recherche | Date de publication | Membre(s) de la famille de brevet(s) | Date de publication |
|---|---|---|---|
| EP 642781 A | 15-03-1995 | FR 2709982 A | 24-03-1995 |
| | | CA 2132144 A | 16-03-1995 |
| | | JP 2693116 B | 24-12-1997 |
| | | JP 7163859 A | 27-06-1995 |
| | | US 5531993 A | 02-07-1996 |
| | | US 5863545 A | 26-01-1999 |
| FR 2698004 A | 20-05-1994 | AT 161708 T | 15-01-1998 |
| | | DE 69316177 D | 12-02-1998 |
| | | DE 69316177 T | 16-04-1998 |
| | | EP 0603019 A | 22-06-1994 |
| | | JP 6211626 A | 02-08-1994 |
| | | US 5368850 A | 29-11-1994 |

EPO FORM P0460

Pour tout renseignement concernant cette annexe : voir Journal Officiel de l'Office européen des brevets, No.12/82